# EUROPEAN PATENT SPECIFICATION

(11) **EP 1 688 398 B1**
(45) Date of publication and mention of the grant of the patent: **16.04.2014**
(21) Application number: 06002148.2
(22) Date of filing: 02.02.2006
(51) Int. Cl.: C03C 10/00, C03C 10/04, A61K 6/06, C03C 3/085, A61K 6/027, C03B 32/02, C03C 4/00

(54) **Lithium silicate glass ceramic**
Lithiumsilikat-Glaskeramik
Vitrocéramique à base du silicate de lithium

(30) Priority: 08.02.2005 EP 05002588; 20.06.2005 DE 102005028637
(43) Date of publication of application: 09.08.2006
(60) Divisional application: 10013129.1
(73) Proprietor: Ivoclar Vivadent AG, 9494 Schaan (LI)
(72) Inventor: Apel, Elke, 9475 Sevelen (CH); Höland, Wolfram Dr. Prof., 9494 Schaan (LI); Schweiger, Marcel, Dipl.-Ing., 7000 Chur (CH); van t' Hoen, Christian, 6710 Nenzing (AT); Bürke, Harald, Dr., 97222 Rimpar (DE); Rheinberger, Volker, Dr., 9490 Vaduz (LI)
(74) Representative: UEXKÜLL & STOLBERG

(56) References cited:
- EP-A- 0 536 572
- EP-A- 0 827 941
- EP-A- 1 505 041
- DE-A1- 2 451 121
- US-A1- 2002 010 063
- US-B1- 6 455 451

## Description

The invention primarily relates to lithium silicate glass ceramic materials which can be easily shaped by machining and subsequently converted into shaped products with high strength.

There is an increasing demand for materials which can be processed into dental restorative products, such as crowns, inlays and bridges, by means of computer controlled milling machines. Such CAD/CAM methods are very attractive as they allow to provide the patient quickly with the desired restoration. A so-called chair-side treatment is thus possible for the dentist.

However, materials suitable for processing via computer aided design / computer aided machining (CAD/CAM) methods have to meet a very specific profile of properties.

First of all, they need to have in the finally prepared restoration appealing optical properties, such as translucence and shade, which imitate the appearance of the natural teeth. They further need to show high strength and chemical durability so that they can take over the function of the natural tooth material and maintain these properties over a sufficient period of time while being permanently in contact with fluids in the oral cavity which can even be aggressive, such as acidic in nature.

Secondly and very importantly, it should be possible to machine them in an easy manner into the desired shape without undue wear of the tools and within short times. This property requires a relatively low strength of the material and is therefore in contrast to the desired properties mentioned above for the final restoration.

The difficulty of combining the properties of low strength in the stage of the material to be processed and a high strength of the final restoration is reflected by the known materials for a CAD/CAM processing which are in particular with respect to an easy machinability unsatisfactory.

DE-A-197 50 794 discloses lithium disilicate glass ceramics which are primarily intended to be shaped to the desired geometry by a hot-pressing process wherein the molten material is pressed in the viscous state. It is also possible for these materials to be shaped by computer aided milling processes. However, it has been shown that the machining of these materials results in a very high wear of the tools and very long processing times. These disadvantages are caused by the high strength and toughness primarily imparted to the materials by the lithium disilicate crystalline phase. Moreover, it has been shown that the machined restorations show only a poor edge strength. The term "edge strength" refers to the strength of parts of the restoration having only a small thickness in the range of few 1/10 mm.

Further approaches of achieving easy machinability together with a high strength of the final restoration have also been made. EP-B-774 993 and EP-B-817 597 describe ceramic materials on the basis of Al₂O₃ or ZrO₂ which are machined in an unsintered state which is also referred to as "green state". Subsequently, the green bodys are sintered to increase the strength. However, these ceramic materials suffer from a drastical shrinkage of up to 50 % by volume (or up to 30 % as linear shrinkage) during the final sintering step. This leads to difficulties in preparing the restorations with exactly the dimensions as desired. The substantial shrinkage represents a particular problem if complicated restorations are manufactured, such as a multi-span bridge.

From S.D. Stookey: "Chemical Machining of Photosensitive Glass", Ind. Eng. Chem., 45, 115-118 (1993) and S.D. Stookey: "Photosensitively Opacifiable Glass" US-A-2 684 911 (1954) it is also known that in lithium silicate glass ceramics a metastable phase can be formed at first. For example in photosensitive glass ceramics (Fotoform^{®}, FotoCeram^{®}) Ag-particles are formed using UV-light. These Ag-particles serve as crystallization agent in a lithium metasilicate phase. The areas which were exposed to light are in a subsequent step washed out by diluted HF. This procedure is possible since the solubility of the lithium metasilicate phase in HF is much higher than the solubility of the parent glass. The glass portion remaining after said solubilizing process (Fotoform^{®}) can be transferred into a lithium disilicate glass ceramic (FotoCeram^{®}) by an additional heat treatment.

Also investigations of Borom, e.g. M.-P. Borom, A.M. Turkalo, R.H. Doremus: "Strength and Microstructure in Lithium Disilicate Glass-Ceramics", J. Am. Ceram. Soc., 58, No. 9-10, 385-391 (1975) and M.-P. Borom, A.M. Turkalo, R.H. Doremus: "Verfahren zum Herstellen von Glaskeramiken" DE-A-24 51 121 (1974), show that a lithium disilicate glass ceramic can in the first instance crystallize in varying amounts as metastable lithium metasilicate phase. However, there also exist compositions which crystallize in the form of the disilicate phase from the beginning and the metasilicate phase is not present at all. A systematic investigation of this effect has not become known. From the investigations of Borom it is also known that the glass ceramic which contains lithium metasilicate as the main phase has a reduced strength compared to the one of a glass ceramic which only contains a lithium disilicate phase.

Document D3 (US 2002/0010063) discloses a process for the preparation of shaped translucent lithium disilicate glass ceramic products, which may be used in particular as dental restoration (abstract). The process involves the preparation of a glass followed by a heat treatment (claim 1) to obtain so-called "blanks" (paragraph 78). The composition of the glass ceramic comprises ZnO.

It has further been found out that the presence of ZnO in lithium silicate glass ceramics of the prior art is undesirable especially when highly translucent dental restorations are to be produced. Under such circumstances, the strong opalescent effect caused by ZnO is apparent and results in unacceptable optical properties for a restoration which is to imitate the natural tooth material.

It is, therefore, an object of the present invention to eliminate these disadvantages and in particular to provide a material which can be easily shaped by computer-aided milling and trimming processes and can subsequently be converted into a high-strength dental product which also displays a high chemical durability and excellent optical properties and exhibits a drastically reduced shrinkage during said final conversion, and achieves all these properties without the need for ZnO as a component.

This object is achieved by the process for preparing a dental restoration according to claims 1 to 23. The invention also relates to the use of a lithium silicate glass ceramic or glass according to claims 24 to 28.

It has surprisingly been shown that by using a starting glass of a very specific composition and a specific process it is possible to provide in particular a glass ceramic which has metastable lithium metasilicate (Li₂SiO₃) as main crystalline phase rather than lithium disilicate (Li₂Si₂O₅). This lithium metasilicate glass ceramic has a low strength and toughness and hence can be easily machined into the shape of even complicated dental restorations, but can after such machining be converted by a heat treatment into a lithium disilicate glass ceramic product with outstanding mechanical properties, excellent optical properties, in particular a strongly reduced opalescence, and very good chemical stability thereby undergoing only a very limited shrinkage.

The lithium silicate glass ceramic used according to the invention comprises the following components:

| Component | wt. -% | |
|---|---|---|
| SiO₂ | 64.0 - 75.0, | in particular 64.0 - 73.0 |
| Li₂O | 13.0 - 17.0 | |
| K₂O | 2.0 - 5.0 | |
| Al₂O₃ | 0.5 - 5.0 | |
| Nucleating agent | 2.0 - 5.0 | |
| Me(II)O | 0 - 3.0 | |

with Me (II) O being selected from at least one of CaO, BaO, MgO and SrO,
and comprises less than 0.1 wt.% of ZnO,
and comprises lithium metasilicate as main crystalline phase.

It is preferred that the glass ceramic is essentially free of ZnO.

It is surprising that even without presence of ZnO the glass ceramic used according to the invention fulfils the mentioned multiple requirements. This was possible by the selection of the other components and their amounts and preferably by the ratios of some of these components to each other.

The glass ceramics used according to the invention comprise lithium metasilicate as main crystalline phase. Such glass ceramics are also referred to in the following as lithium metasilicate glass ceramics.

It has also been found out to be beneficial if the glass ceramic comprises 0 to 2.0 and preferably 0 to 1.5 wt.% of Me(II)O. Me(II)O is in particular selected from at least one of CaO and MgO. Particularly preferred glass ceramics comprise 0.1 to 1.0 wt.% of MgO.

The nucleating agent is preferably at least one of P₂O₅ and compounds of the elements Pt, Ag, Cu and W. It serves to induce formation of lithium metasilicate crystalls and is preferably P₂O₅.

Further, it was shown that a specific molar ratio of SiO₂ to Li₂O serves to ensure that upon the necessary heat treatment of a corresponding starting glass mainly lithium metasilicate and lithium disilicate, respectively, is produced. This is of particular importance. While a lithium metasilicate glass ceramic essentially free of lithium disilicate results in particular in an excellent machinability, a lithium disilicate restoration essentially free of the easily dissolvable lithium metasilicate has a very good chemical stability.

Thus, it was found preferable that the molar ratio of SiO₂ : Li₂O is at least 2.2 : 1, preferably at least 2.3 : 1, and most preferred in the range of 2.3 : 1 to 2.5 : 1.

Moreover, investigations revealed that the molar ratio of Al₂O₃ : K₂O is of significance for obtaining the desired tranclucence and the predominant crystallization of lithium metasilicate.

It is prefered that the molar ratio of Al₂O₃ : K₂O is in the range of 1 : 0.5 to 1 : 2.0 and preferably is from 1 : 1 to 1 : 2.0.

There also exist preferred ranges for the amounts of components of the glass ceramic used according to the invention. These can be used independently from each other.

It is preferred that the glass ceramic comprises 2.5 to 5.0 wt.% of Al₂O₃.

It is also preferred that the glass ceramic comprises 70.0 to 73.0 wt.% of SiO₂.

It is also preferred that the glass ceramic comprises 0 to 4.0, preferably 0.1 to 4.0, more preferably 1.0 to 4.0 and most preferred 1.5 to 3.0 wt.% of ZrO₂. If the emphasis is on the achieving of a high strength of the final lithium disilicate ceramic, then 0 to 2.0 wt.% to of ZrO₂ are advantageous.

It is further preferred that the glass ceramic comprises at least one of the following components in an amount of:

| Component | wt. -% | |
|---|---|---|
| Li₂O | 14.0 - 16.0 | |
| K₂O | 3.0 - 4.5 | |
| coloring and fluorescent metal oxides | 0 - 7.5, | preferably 0.5 - 3.5. |

The metal of the coloring and fluorescent metal oxides is preferably selected from group f-elements and in particular from the group of Ta, Tb, Y, La, Er, Pr, Ce, Ti, V, Fe and Mn. The colouring or fluorescent oxides ensure that the colour of the final dental product matches that of the natural tooth material of the patient in question.

Further, the glass ceramic may comprise as additional component Na₂O in an amount of 0 to 2.0 wt.-%.

Additional components to enhance the technical processability of the glass may also be present. Such additional components may therefore be in particular compounds such as B₂O₃ and F which in general amount to 0 to 5.0 % by weight.

Generally the amount of lithium metasilicate is 20 to 80 vol.-%. It has surprisingly been shown that a specific volume portion of lithium metasilicate should be present to achieve excellent processing properties. Thus, it is further preferred that the lithium metasilicate crystalline phase forms 20 to 50 vol% and in particular 30 to 40 vol% of the lithium silicate glass ceramic. Such a part of the volume leads to the crystals being present rather remote from each other and hence avoids a too high strength of the glass ceramic.

If the emphasis is on the achieving of a high strength of the lithium disilicate ceramic, then the lithium metasilicate phase preferably forms more than 50 and up to 80 vol.% of the lithium silicate glass ceramic.

The lithium metasilicate crystals are preferably of lamellar or platelet form. This leads to a very good machinability of the lithium metasilicate glass ceramic without use of high energy and without uncontrolled breaking. The latter aspect of uncontrolled breaking is for example known from glasses which are generally unsuitable for machining. It is assumed that the preferred morphology of the lithium metasilicate crystals is also responsible for the surprisingly high edge strength of products, e.g. complicated dental restorations, can be made from the lithium metasilicate glass ceramic used according to the invention.

The lithium silicate glass ceramic used according to the invention preferably is in the form of a blank. The blank usually takes the form of a small cylinder or a rectangular block. The exact form depends on the specific apparatus used for the desired computer-aided machining of the blank.

After the machining, the lithium silicate glass ceramic has preferably the shape of a dental restoration, such as an inlay, an onlay, a bridge, an abutment, a facing, a veneer, a facet, a crown, a partial crown, a framework or a coping.

A lithium silicate glass ceramic which comprises lithium disilicate as main crystalline phase can be formed in a process wherein the lithium metasilicate of a glass ceramic used according to the invention is converted to lithium disilicate crystals.

A dental product made from lithium disilicate glass ceramic can be formed in a process wherein the lithium metasilicate of a glass ceramic used according to the invention is converted to lithium disilicate crystals.

The lithium metasilicate glass ceramic used according to the invention is preferably prepared by a process which comprises
(a) producing a starting glass containing the components of the glass ceramic,
(b) subjecting the starting glass to a first heat treatment at a first temperature to give a glass product which contains nuclei suitable for forming lithium metasilicate crystals,
(c) subjecting the glass product to a second heat treatment at a second temperature which is higher than the first temperature to obtain the lithium silicate glass ceramic with lithium metasilicate as the main crystalline phase.

In step (a), usually a melt of a starting glass is produced which contains the components of the glass ceramic. For this purpose a corresponding mixture of suitable starting materials, such as carbonates, oxides, and phosphates, is prepared and heated to temperatures of, in particular 1300 to 1600°C, for 2 to 10 hours. In order to obtain a particularly high degree of homogeneity, the glass melt obtained may be poured into water to form glass granules and the glass granules obtained are melted again.

It further preferred that the melt of the starting glass is cooled, such as to room temperature, before subjecting it to step (b). The melt of the starting glass is also usually poured into a mould to form a starting glass blank.

In some cases it is convenient to control a cooling procedure such that it not only relaxes the glass, but also effects the first heat treatment of step (b).

In step (b) the starting glass is subjected to a first heat treatment at a first temperature to cause formation of nuclei for lithium metasilicate crystals. Preferably, this first heat treatment comprises heating the starting glass to a temperature of 500 to 600°C for a period of about 10 minutes to 3 hours. This results in formation of a great number of nuclei that ensure a very satifactory crystal growth. It also ensures that in the further processing after step (c) to give a lithium disilicate glass ceramic a very homogeneous lithium disilicate structure can be obtained.

It is also advantageous that the second heat treatment in step (c) comprises heating the glass product to a second temperature of 570° to 750°C, preferably 570 to 670 °C, and more preferably to about 650 °C.

It has further surprisingly been shown that relatively high temperatures lead to high amounts of lithium metasilicate which in turn lead to a high amount of lithium disilicate in the third heat treatment. Such high amounts of lithium disilicate impart a high strength to the ceramic. Thus, if the emphasis is on the achieving a high strength final product, then it is advantageous to carry out the second heat treatment at 680° to 720°C, and preferably 690° to 710°C and more preferably about 700°C.

Depending on the specific composition of a selected starting glass, it is possible for the skilled person by means of differential scanning calorimetry (DSC) and x-ray diffraction analyses to determine suitable conditions in steps (b) and (c) to result in glass ceramics having the desired morphology and size of the crystals of lithium metasilicate. Moreover, these analyses allow also the identification of conditions avoiding or limiting the formation of undesirable other crystalline phases, such as of the high-strength lithium disilicate, or of cristobalite and lithium phosphate.

Usually, the starting glass of step (a), the glass product of step (b), or preferably the lithium metasilicate glass ceramic of step (c) is shaped to a desired geometry by machining or by hot pressing. The machining is in particular performed by grinding, trimming or milling and preferably controlled by a computer using CAD/CAM-based milling devices. This allows a so-called chair-side treatment of the patient by the dentist.

It is a particular advantage of the lithium metasilicate glass ceramic used according to the invention that it can be shaped by machining without the undue wear of the tools observed with the tough and high-strength prior art materials. This is in particular shown by the easy possibility to polish and trim the glass ceramics used according to the invention. Such polishing and trimming processes therefore require less energy and less time to prepare an acceptable product having the form of even very complicated dental restorations.

Further, the lithium metasilicate glass ceramic used according to the invention can advantageously be processed to a lithium disilicate glass ceramic of high strength, which usually has a content of 50 to 85 vol.% and preferably 65 to 80 vol.% of crystalline lithium disilicate phase.

This is preferably effected by a process wherein the prepared lithium metasilicate glass ceramic of step (c) is subjected to a third heat treatment at a third temperature of 830 to 880 °C for a period of 10 to 60 minutes. This heat treatment can also be effected when hot-pressing the lithium metasilicate glass ceramic to achieve a shaping.

Thus, the lithium metasilicate glass ceramic can be further processed to the lithium dilsilicate glass ceramic of desired shape e.g. by both (i) CAD/CAM and a heat treatment or (ii) a hot-pressing. This is very advantageous for the user.

It is also possible to use for these purposes a corresponding lithium silicate glass which comprises nuclei suitable for formation of lithium metasilicate crystals. This glass is a precursor of the lithium metasilicate glass ceramic and the lithium disilicate glass ceramic used according to the invention. The invention is also directed to the use of such a glass. It is obtainable by the above process in step (b). This lithium silicate glass used according to the invention comprises the following components:

| Component | wt. -% | |
|---|---|---|
| SiO₂ | 64.0 to 75.0, | in particular 64.0 - 73.0 |
| Li₂O | 13.0 - 17.0 | |
| K₂O | 2.0 - 5.0 | |
| Al₂O₃ | 0.5 - 5.0 | |
| Nucleating agent | 2.0 - 5.0 | |
| Me(II)O | 0 - 3.0 | |

with Me(II)O being selected from at least one of CaO, BaO, MgO and SrO,
and comprises less than 0.1 wt.% of ZnO, and comprises nuclei suitable for formation of lithium metasilicate crystals.

For manufacturing a dental restoration by the hot pressing technique, it is preferred to use a lithium silicate glass ingot used according to the invention having nuclei for lithium metasilicate. This ingot is heated to about 700 to 1200°C to convert it into a viscous state. The heat treatment can be conducted in a special furnace (EP 500^{®}, EP 600^{®}, Ivoclar Vivadent AG). The ingot is embedded in a special investment material. During the heat treatment the ingot will be crystallized. The main crystal phase is then lithium disilicate. The viscous glass ceramic flows under a pressure of 1 to 4 MPa into the cavity of the investment material to obtain the desired shape of the dental restoration. After cooling the investment mould to room temperature the lithium disilicate restoration can be divested by sand blasting. The restoration can be further coated with a glass or a glass ceramic by sintering or a hot pressing technique to obtain the finalized dental restoration with natural aesthetics.

The same hot-pressing technique can be applied to the lithium metasilicate glass ceramic used according to the invention which will be converted to lithium disilicate glass ceramic.

A preferred method for converting the lithium metasilicate glass ceramic used according to the invention to a lithium disilicate glass ceramic dental restoration by the CAD/CAM technique uses lithium metasilicate glass ceramic blanks, e.g. blocks, having a strength of about 80 to 150 MPa. These can be easily machined in a CAM unit like Cerec 2^{®} or Cerec 3^{®} (Sirona, Germany). Larger milling machines such as DCS precimill^{®} (DCS, Switzerland) are also suitable. The block is therefore positioned in the grinding chamber by a fixed or integrated holder. The CAD construction of the dental restoration is done by a scanning process or an optical camera in combination with a software tool. The milling process needs for one unit about 10 to 15 minutes. Copy milling units such as Celay® (Celay, Switzerland) are also suitable for machining the blocks. First, a 1:1 copy of the desired restoration is fabricated in hard wax. The wax model is then mechanically scanned and 1:1 mechanically transmitted to the grinding tool. The grinding process is therefore not controlled by a computer. The milled dental restoration has to be subjected to the third heat treatment to obtain the desired lithium disilicate glass ceramic with high strength and tooth-like color. The product can be further coated with a glass or a glass ceramic by sintering or hot pressing technique to obtain the final dental restoration with natural aesthetics.

The lithium metasilicate glass ceramic used according to the invention can also be used for coating a dental restoration. The coating is preferably effected by hot-pressing the lithium metasilicate glass ceramic onto the restoration.

It was surprisingly found that the easily machinable lithium metasilicate glass ceramic used according to the invention can be converted by a further heat treatment into a lithium disilicate glass ceramic product having also excellent optical properties. The conversion to a lithium disilicate glass ceramic is associated with a very small linear shrinkage of only about 0.2 to 0.3 %, which is almost negligible in comparison to a linear shrinkage of up to 30 % when sintering ceramics. The obtained lithium disilicate glass ceramic has not only excellent mechanical properties, such as high strength, but also displays other properties required for a material for dental restorations. It is emphasized that these properties are achieved without the need for ZnO as a component which may be detrimental for specific restorations in view of its strong opalescent effect.

Thus, a product is finally obtained which has all the beneficial mechanical, optical and stability properties making lithium disilicate ceramics attractive for use as dental restorative materials. However, these properties are achieved without the disadvantages of the conventional materials when shaped by using a CAD/CAM based process, in particular the undue wear of the milling and trimming tools.

The invention is explained in more detail below on the basis of Examples.

### Examples

### Examples 1 to 8

A total of 8 different lithium metasilicate glass ceramics used according to the invention with the chemical compositions given in Table I were prepared using the indicated second heat treatment. The obtained glass ceramics were then converted to the corresponding lithium disilicate glass ceramics using the indicated third heat treatment.

Firstly, samples of the corresponding starting glasses were melted in a platinum-rhodium crucible at a temperature of 1450 °C and for a period of 40 minutes. The glass melt was poured into water and the obtained granules were, after drying, again melted at 1500°C. The glass melts obtained were then poured into graphite moulds to give blocks. After relaxation of the glass blocks at 500 to 600°C for 10 minutes to 3 hours, they were subjected to the given second heat treatment. Before effecting the third heat treatment, the blocks were checked for their machinability by milling in a CAD-CAM milling machine (i.e. CEREC 3^{®}). Finally, the indicated third heat treatment was conducted. The crystal phases present after the second and third heat treatment were identified by XRD techniques and are given in table I.

Further, the opalescence of the products was visually assessed and the contrast value CR was determined according to BS 5612 (British Standard) using a spectral colorimeter (Minolta CM-3700d). The chemical stability in acetic acid was determined as well as the stability in artificial saliva. The corresponding data are to be found in the following Table II and show in particular the surprising combination of a lack of opalescence together with a high translucence and stability. The composition of the artificial saliva is given in table III.

The data obtained show that the lithium metasilicate glass ceramics used according to the invention combine a very good machinability and high edge strength with the easy possibility to convert them by a simple heat treatment into lithium disilicate glass ceramics which have a very high bending strength as well as an excellent chemical durability and good translucence, all of which being properties which make them very attractive as materials useful for the manufacture of dental restorations.

### Examples 9 to 12

Four glass ceramics used according to the invention were prepared in analogous manner as examples 1 to 8. However, the heat treatment scheme was different. In addition each material was subjected to the schemes referred to as "Cycle A" and "Cycle B" which differ in the temperature used for the crystallization of lithium metasilicate, namely 650° and 700°C, respectively.

Details as to the materials prepared and tested as well as their properties are given in the table IV. It is apparent that the "Cycle B" treatment using a temperature of 700°C for the crystallization of lithium metasilicate leads to lithium disilicate glass ceramics having excellent strengths.

**Table I**

| **Example** | **1** | **2** | **3** | **4** | **5** | **6** | **7** | **8** |
|---|---|---|---|---|---|---|---|---|
| **Molar ratio** | | | | | | | | |
| **SiO2:Li2O** | **2.39:1** | **2.39:1** | **2.4:1** | **2.39:1** | **2.39:1** | **2.39:1** | **2.39:1** | **2.39:1** |
| **Al2O3:K2O** | **1:1.0** | **1:1.0** | **1:1.2** | **1:1.20** | **1:1.35** | **1:1.50** | **1:1.70** | **1:1.30** |
| | | | | | | | | |

| | **wt.-% (Mol%)** | **wt.-% (Mol%)** | **wt.-% (Mol%)** | **wt.-% (Mol%)** | **wt.-% (Mol%)** | **wt.-% (Mol%)** | **wt.-% (Mol%)** | **wt.-% (Mol%)** |
|---|---|---|---|---|---|---|---|---|
| **SiO2** | 72.21 (66.12) | 70.64 (65.62) | 70.52 (65.52) | 70.78 (65.57) | 70.78 (65.56) | 70.78 (65.56) | 70.78 (65.55) | 70.78 (65.29) |
| **K2O** | 3.16 (1.85) | 3.09 (1.83) | 3.81 (2.26) | 3.76 (2.22) | 3.96 (2.34) | 4.16 (2.46) | 4.36 (2.58) | 3.36 (1.98) |
| **Li2O** | 14.99 (27.60) | 14.68 (27.43) | 14.64 (27.35) | 14.7 (27.38) | 14.7 (27.38) | 14.7 (27.37) | 14.7 (27.37) | 14.7 (27.26) |
| **Al2O3** | 3.45 (1.86) | 3.38 (1.85) | 3.35 (1.83) | 3.38 (1.85) | 3.18 (1.74) | 2.98 (1.63) | 2.78 (1.52) | 2.78 (1.51) |
| **P2O5** | 3.28 (1.27) | 3.21 (1.26) | 3.2 (1.26) | 3.21 (1.26) | 3.21 (1.26) | 3.21 (1.26) | 3.21 (1.26) | 3.21 (1.25) |
| **ZrO2** | 2.91 (1.30) | 3.00 (1.36) | 2.5 (1.13) | 1.8 (0.81) | 1.8 (0.81) | 1.80 (0.81) | 1.8 (0.81) | 1.8 (0.81) |
| **CeO2** | | 1.88 (0.61) | 1.86 (0.60) | 2.00 (0.65) | 2.00 (0.65) | 2.00 (0.65) | 2.00 (0.65) | 2.00 (0.65) |
| **V2O5** | | 0.12 (0.04) | 0.12 (0.04) | 0.07 ((0.02) | 0.07 ((0.02) | 0.07 ((0.02) | 0.07 (0.02) | 0.07 (0.02) |
| **MnO2** | | | | 0.03 (0.02) | 0.03 (0.02) | 0.03 (0.02) | 0.03 (0.02) | 0.03 (0.02) |
| **Er2O3** | | | | 0.12 (0.017) | 0.12 (0.017) | 0.12 (0.017) | 0.12 (0.017) | 0.12 (0.017) |
| **MgO** | | | | 0.15 (0.21) | 0.15 (0.21) | 0.15 (0.21) | 0.15 (0.21) | 0.15 (0.21) |
| **CaO** | | | | | | | | 1.00 (0.99) |

| **Crystalline phases after:** | | | | | | | | |
|---|---|---|---|---|---|---|---|---|
| **Second heat treatment: 20'/650°C** | Li2SiO3 | Li2SiO3 | Li2SiO3 | Li2SiO3 | | Li2SiO3 | Li2SiO3 | Li2SiO3 |
| | Li2Si2O5* | Li2Si2O5* | | | | | | Li2Si2O5* |
| **Third heat treatment: 10'/850°C** | Li2Si2O5 | Li2Si2O5 | Li2Si2O5 | Li2Si2O5 | Li2Si2O5 | Li2Si2O5 | Li2Si2O5 | Li2Si2O5 |
| | Li3PO4* | Li3PO4* | Li3PO4* | Li3PO4* | Li3PO4* | li3PO4* | Li3PO4* | Li3PO4* |

**Table II**

| Example | 1 | 2 | 3 | 4 | 6 |
|---|---|---|---|---|---|
| CR-Value BS-5612 (1978) | 40.4 | 37.0 | 50.0 | 59.3 | 58.8 |
| Opalescence | No | No | No | No | No |
| Chemical stability in Acetic acid (24h/ 80°C, mass loss in µg/cm²) | 9 | 18 | 48 | 3 | 9 |
| Chemical stability in Saliva (7d/60°C, mass loss in µg/cm²) | 13 | 17 | 28 | 27 | 17 |

**Table III:**

| | Composition of artificial saliva |
|---|---|
| Component | Amount in mg in a total of 500 ml H₂O |
| NaCl | 125.64 |
| KCl | 963.9 |
| NH₄Cl | 178.0 |
| CaCl₂ · 2H₂O | 227.8 |
| KSCN | 189.2 |
| CO(NH2)₂ | 200.0 |
| Na₂SO₄ · 10H₂O | 336.2 |
| NaHCO₃ | 630.8 |
| KH₂PO₄ | 654.5 |

**Table IV**

| Example | 9 | 10 | 11 | 12 |
|---|---|---|---|---|
| | | | | |
| **SiO₂** | 74.37 | 72.89 | 72.21 | 71.40 |
| **K₂O** | 3.26 | 3.18 | 3.16 | 3.13 |
| **Li₂O** | 15.44 | 15.13 | 14.99 | 14.79 |
| **Al₂O₃** | 3.55 | 3.48 | 3.45 | 3.41 |
| **P₂O₅** | 3.38 | 3.31 | 3.28 | 3.22 |
| **ZrO₂** | 0.00 | 2.01 | 2.91 | 4.05 |
| | All values above in wt.-% | | | |
| **SiO₂:Li₂O (Mol.-ratio%)** | **2.39** | **2.40** | **2.39** | **2.40** |
| | | | | |

| **Cycle A:** | (1) 500°C/ 10 min + (2) 650°C/ 20 min + (3) 850°C/10min*) | | | |
|---|---|---|---|---|
| **Biaxial Flexural Strength /MPa** | 786 +/- 92 | 515 +/- 54 | 522 +/- 82 | 479 +/- 36 |
| **Contrast Ratio** | 0.80 | 0.56 | 0.43 | 0.36 |
| | | | | |

| **cycle B:** | (1) 500°C/ 10 min + (2) 700°C/ 20 min + (3) 850°C/10min *) | | | |
|---|---|---|---|---|
| **Biaxial Flexural Strength /MPa** | 828 +/- 104 | 659 +/- 75 | 608 +/- 90 | 694 +/- 113 |
| **Contrast Ratio** | 0.83 | 0.63 | 0.53 | 0.41 |
| ***)** | (1) Nucleation in the glass | | | |
| | (2) Crystallization of Li-Metasilicate | | | |
| | (3) Crystallization of Li-Disilicate from Li-Metasilicate | | | |

## Claims

1. Process for preparing a dental restoration, wherein a lithium silicate glass ceramic is used which comprises the following components:
| Component | wt. -% | |
|---|---|---|
| SiO₂ | 64.0 - 75.0, | preferably 64.0 - 73.0 |
| Li₂O | 13.0 - 17.0 | |
| K₂O | 2.0 - 5.0 | |
| Al₂O₃ | 0.5 - 5.0 | |
| Nucleating agent | 2.0 - 5.0 | |
| Me(II)O | 0 - 3.0 | |
and which comprises less than 0.1 wt.% of ZnO,
with Me (II) O being selected from at least one of CaO, BaO, MgO and SrO,
and which comprises lithium metasilicate as main crystalline phase.

2. Process according to claim 1, wherein the glass ceramic is essentially free of ZnO.

3. Process according to claim 1 or 2, wherein the glass ceramic comprises 0 to 2.0 and preferably 0 to 1.5 wt.% of Me(II)O.

4. Process according to any one of claims 1 to 3, wherein Me(II)O is selected from at least one of CaO and MgO.

5. Process according to any one of claims 1 to 4, wherein the glass ceramic comprises 0.1 to 1.0 wt.% of MgO.

6. Process according to any one of claims 1 to 5, wherein the molar ratio of SiO₂ : Li₂O is at least 2.2 : 1, preferably at least 2.3 : 1, and most preferred in the range of 2.3 : 1 to 2.5 : 1.

7. Process according to any one of claims 1 to 6, wherein the molar ratio of Al₂O₃ : K₂O is in the range of 1 : 0.5 to 1 : 2.0 and preferably 1 : 1 to 1 : 2.0.

8. Process according to any one of claims 1 to 7, wherein the glass ceramic comprises 2.5 to 5.0 wt.% of Al₂O₃.

9. Process according to any one of claims 1 to 8, wherein the glass ceramic comprises 70.0 to 73.0 wt.% of SiO₂.

10. Process according to any one of claims 1 to 9, wherein the glass ceramic comprises 0 to 4.0, preferably 0.1 to 4.0, more preferably 1.0 to 4.0 and most preferred 1.5 to 3.0 wt.% of ZrO₂.

11. Process according to any one of claims 1 to 10, wherein the glass ceramic comprises at least one of the following components in an amount of:
| Component | wt. -% | |
|---|---|---|
| Li₂O | 14.0 - 16.0 | |
| K₂O | 3.0 - 4.5 | |
| coloring and fluorescent metal oxides | 0.5 - 7.5, | preferably 0.5 - 3.5. |

12. Process according to any one of claims 1 to 11, wherein the glass ceramic further comprises at least one of the following additional components
| Component | wt. -% |
|---|---|
| Na₂O | 0 - 2.0 |
| B₂O₃ | 0 - 5.0 |
| F | 0 - 5.0. |

13. Process according to any one of claims 1 to 12, wherein the nucleating agent is at least one of P₂O₅ and compounds of the elements Pt, Ag, Cu and W.

14. Process according to any one of claims 1 to 13, wherein the lithium metasilicate forms more than 50 and up to 80 vol.-% of the lithium silicate glass ceramic.

15. Process according to any one of claims 1 to 14, wherein the glass ceramic is in form of a blank.

16. Process according to any one of claims 1 to 15, wherein the dental restoration is an inlay, an onlay, a bridge, an abutment, a facing, a veneer, a facet, a crown, a partial crown, a framework or a coping.

17. Process according to any one of claims 1 to 16, wherein the dental restoration comprises lithium disilicate as main crystalline phase.

18. Process according to any one of claims 1 to 17, wherein the lithium metasilicate glass ceramic is prepared by
(a) producing a starting glass containing the components of the glass ceramic,
(b) subjecting the starting glass to a first heat treatment at a first temperature to give a glass product which contains nuclei suitable for forming lithium metasilicate crystals,
(c) subjecting the glass product to a second heat treatment at a second temperature which is higher than the first temperature to obtain the lithium silicate glass ceramic with lithium metasilicate as the main crystalline phase.

19. Process according to claim 18, wherein the first heat treatment in step (b) comprises heating the starting glass to a temperature of 500 to 600°C for a period of about 10 minutes to 3 hours.

20. Process according to claim 18 or 19, wherein the second heat treatment in step (c) comprises heating the glass product to a second temperature of 680° to 720°C, preferably 690 to 710°C, and more preferably to about 700°C.

21. Process according to any one of claims 18 to 20, wherein the starting glass of step (a), the glass product of step (b), or the lithium metasilicate glass ceramic of step (c) is shaped to a desired geometry by machining or by hot pressing.

22. Process according to claim 21, wherein the machining is performed by grinding, trimming or milling.

23. Process according to any one of claims 18 to 22, which further comprises subjecting the lithium silicate glass ceramic of step (c) to a third heat treatment at a third temperature of 830 to 880 °C for a period of 10 to 60 minutes.

24. Use of the glass ceramic defined in any of claims 1 to 15 for the preparation of a dental restoration.

25. Use of a lithium silicate glass ceramic for the preparation of a dental restoration, wherein the lithium silicate glass ceramic comprises the following components:
| Component | wt. -% |
|---|---|
| SiO₂ | 64.0 - 73.0 |
| Li₂O | 13.0 - 17.0 |
| K₂O | 2.0 - 5.0 |
| Al₂O₃ | 0.5 - 5.0 |
| Nucleating agent | 2.0 - 5.0 |
| Me(II)O | 0 - 1.5 |
wherein the molar ratio of Al₂O₃ : K₂O is in the range of 1 : 0.5 to 1 : 2.0,
with Me(II)O being selected from at least one of CaO, BaO, MgO and SrO,
and comprises less than 0.1 wt.% of ZnO.

26. Use of a lithium silicate glass for the preparation of a dental restoration, wherein the lithium silicate glass comprises the following components:
| Component | wt. -% |
|---|---|
| SiO₂ | 64.0 - 73.0 |
| Li₂O | 13.0 - 17.0 |
| K₂O | 2.0 - 5.0 |
| Al₂O₃ | 0.5 - 5.0 |
| Nucleating agent | 2.0 - 5.0 |
| Me(II)O | 0 - 1.5 |
wherein the molar ratio of Al₂O₃ : K₂O is in the range of 1 : 0.5 to 1 : 2.0,
with Me(II)O being selected from at least one of CaO, BaO, MgO and SrO,
and comprises less than 0.1 wt.% of ZnO, and comprises nuclei suitable for formation of lithium metasilicate crystals.

27. Use according to claim 26, wherein the molar ratio of SiO₂
: Li₂O is at least 2.2 : 1, preferably at least 2.3 : 1, and most preferred in the range of 2.3 : 1 to 2.5 : 1.

28. Use according to claim 26 or 27, wherein the molar ratio of Al₂O₃ : K₂O is in the range of 1 : 1 to 1 : 2.0.

## Patentansprüche

1. Verfahren zur Herstellung einer dentalen Restauration, bei dem eine Lithiumsilicat-Glaskeramik verwendet wird, die die folgenden Komponenten enthält:
| Komponente | Gew.-% | |
|---|---|---|
| SiO2 | 64,0 - 75,0, | vorzugsweise 64,0 - 73,0 |
| Li₂O | 13,0 - 17,0 | |
| K₂O | 2,0 - 5,0 | |
| Al₂O₃ | 0,5 - 5,0 | |
| Keimbildungsmittel | 2,0 - 5,0 | |
| Me(II)O | 0 - 3,0, | |
und die weniger als 0,1 Gew.-% ZnO enthält,
wobei Me(II)O aus mindestens einem von CaO, BaO, MgO und SrO ausgewählt ist,
und die Lithiummetasilicat als Hauptkristallphase enthält.

2. Verfahren nach Anspruch 1, bei dem die Glaskeramik im wesentlichen frei von ZnO ist.

3. Verfahren nach Anspruch 1 oder 2, bei dem die Glaskeramik 0 bis 2,0 und vorzugsweise 0 bis 1,5 Gew.-% Me(II)O enthält.

4. Verfahren nach einem der Ansprüche 1 bis 3, bei dem Me(II)O ausgewählt ist aus mindestens einem von CaO und MgO.

5. Verfahren nach einem der Ansprüche 1 bis 4, bei dem die Glaskeramik 0,1 bis 1,0 Gew.-% MgO enthält.

6. Verfahren nach einem der Ansprüche 1 bis 5, bei dem das molare Verhältnis von SiO₂ : Li₂O mindestens 2,2:1, vorzugsweise mindestens 2,3:1, und besonders bevorzugt im Bereich von 2,3:1 bis 2,5:1 ist.

7. Verfahren nach einem der Ansprüche 1 bis 6, bei dem das molare Verhältnis von Al₂O₃ : K₂O im Bereich von 1:0,5 bis 1:2,0 und vorzugsweise 1:1 bis 1:2,0 ist.

8. Verfahren nach einem der Ansprüche 1 bis 7, bei dem die Glaskeramik 2,5 bis 5,0 Gew.-% Al₂O₃ enthält.

9. Verfahren nach einem der Ansprüche 1 bis 8, bei dem die Glaskeramik 70,0 bis 73,0 Gew.-% SiO₂ enthält.

10. Verfahren nach einem der Ansprüche 1 bis 9, bei dem die Glaskeramik 0 bis 4,0, vorzugsweise 0,1 bis 4,0, besonders bevorzugt 1,0 bis 4,0 und ganz besonders bevorzugt 1,5 bis 3,0 Gew.-% ZrO₂ enthält.

11. Verfahren nach einem der Ansprüche 1 bis 10, bei dem die Glaskeramik mindestens eine der folgenden Komponenten enthält in einer Menge von:
| Komponente | Gew.-% | |
|---|---|---|
| Li₂O | 14,0 - 16,0 | |
| K₂O | 3,0 - 4,5 | |
| färbende und fluoreszierende Metalloxide | 0,5 - 7,5, | vorzugsweise 0,5 - 3,5 |

12. Verfahren nach einem der Ansprüche 1 bis 11, bei dem die Glaskeramik außerdem mindestens eine der folgenden zusätzlichen Komponenten enthält
| Komponente | Gew.-% |
|---|---|
| Na₂O | 0 - 2,0 |
| B₂O₃ | 0 - 5,0 |
| F | 0 - 5,0. |

13. Verfahren nach einem der Ansprüche 1 bis 12, bei dem das Keimbildungsmittel mindestens eines von P₂O₅ und Verbindungen der Elemente Pt, Ag, Cu und W ist.

14. Verfahren nach einem der Ansprüche 1 bis 13, bei dem das Lithiummetasilicat mehr als 50 und bis zu 80 Vol-% der Glaskeramik bildet.

15. Verfahren nach einem der Ansprüche 1 bis 14, bei dem die Glaskeramik in Form eines Rohlings vorliegt.

16. Verfahren nach einem der Ansprüche 1 bis 15, bei dem die dentale Restauration ein Inlay, ein Onlay, eine Brücke, ein Stiftaufbau, eine Verblendung, eine Schale, eine Facette, eine Krone, eine Teilkrone, ein Gerüst oder eine Kappe ist.

17. Verfahren nach einem der Ansprüche 1 bis 16, bei dem die Dentalrestauration Lithiumdisilicat als Hauptkristallphase enthält.

18. Verfahren nach einem der Ansprüche 1 bis 17, bei dem die Lithiummetasilikat-Glaskeramik hergestellt wird, indem
(a) ein Ausgangsglas hergestellt wird, das die Komponenten der Glaskeramik enthält,
(b) das Ausgangsglas einer ersten Wärmebehandlung bei einer ersten Temperatur unterworfen wird, um ein Glasprodukt zu ergeben, welches Keime enthält, die zur Bildung von Lithiummetasilicatkristallen geeignet sind,
(c) das Glasprodukt einer zweiten Wärmebehandlung bei einer zweiten Temperatur unterworfen wird, die höher als die erste Temperatur ist, um die Lithiumsilicat-Glaskeramik mit Lithiummetasilicatkristallen als die Hauptkristallphase zu erhalten.

19. Verfahren nach Anspruch 18, bei dem die erste Wärmebehandlung in Schritt (b) das Erwärmen des Ausgangsglases auf eine Temperatur von 500 bis 600°C für eine Dauer von etwa 10 Minuten bis 3 Stunden beinhaltet.

20. Verfahren nach Anspruch 18 oder 19, bei dem die zweite Wärmebehandlung in Schritt (c) das Erwärmen des Glasproduktes auf eine zweite Temperatur von 680 bis 720°C, vorzugsweise 690 bis 710°C und besonders bevorzugt auf etwa 700°C beinhaltet.

21. Verfahren nach einem der Ansprüche 18 bis 20, bei dem das Ausgangsglas von Schritt (a), das Glasprodukt von Schritt (b) oder die Lithiummetasilicat-Glaskeramik von Schritt (c) zu einer gewünschten Geometrie durch maschinelle Verarbeitung oder durch Heißpressen verformt wird.

22. Verfahren nach Anspruch 21, bei dem die maschinelle Verarbeitung durch Beschleifen oder Fräsen durchgeführt wird.

23. Verfahren nach einem der Ansprüche 18 bis 22, bei dem außerdem die Lithiumsilicat-Glaskeramik von Schritt (c) einer dritten Wärmebehandlung bei einer dritten Temperatur von 830 bis 880°C für eine Dauer von 10 bis 60 Minuten unterworfen wird.

24. Verwendung der in einem der Ansprüche 1 bis 15 definierten Glaskeramik zur Herstellung einer dentalen Restauration.

25. Verwendung einer Lithiumsilikat-Glaskeramik zur Herstellung einer dentalen Restauration, wobei die Lithiumsilicat-Glaskeramik die folgenden Komponenten enthält:
| Komponente | Gew.-% |
|---|---|
| SiO₂ | 64,0 - 73,0 |
| Li₂O | 13,0 - 17,0 |
| K₂O | 2,0 - 5,0 |
| Al₂O₃ | 0,5 - 5,0 |
| Keimbildungsmittel | 2, 0 - 5,0 |
| Me(II)O | 0 - 3,0, |
wobei das molare Verhältnis von Al₂O₃ : K₂O im Bereich von 1:0,5 bis 1:2,0 ist,
wobei Me(II)O ausgewählt ist aus mindestens einem von CaO, BaO, MgO und SrO,
und die weniger als 0,1 Gew.-% ZnO enthält.

26. Verwendung eines Lithiumsilicat-Glases zur Herstellung einer dentalen Restauration, wobei das Lithiumsilicat-Glas die folgenden Komponenten enthält:
| Komponente | Gew.-% |
|---|---|
| SiO₂ | 64,0 - 73,0 |
| Li₂O | 13,0 - 17,0 |
| K₂O | 2,0 - 5,0 |
| Al₂O₃ | 0,5 - 5,0 |
| Keimbildungsmittel | 2,0 - 5,0 |
| Me(II)O | 0 - 3,0, |
wobei das molare Verhältnis von Al₂O₃ : K₂O im Bereich von 1:0,5 bis 1:2,0 ist,
wobei Me(II)O ausgewählt ist aus mindestens einem von CaO, BaO, MgO und SrO,
und das weniger als 0,1 Gew.-% ZnO enthält und Keime enthält, die zur Bildung von Lithiummetasilicat-Kristallen geeignet sind.

27. Verwendung nach Anspruch 26, wobei das molare Verhältnis von SiO₂ : Li₂O mindestens 2,2:1, vorzugsweise mindestens 2,3:1, und besonders bevorzugt im Bereich von 2,3:1 bis 2,5:1 ist.

28. Verwendung nach Anspruch 26 oder 27, wobei das molare Verhältnis von Al₂O₃ : K₂O im Bereich von 1:1 bis 1:2,0 ist.

## Revendications

1. Procédé de préparation d'une restauration dentaire, dans lequel une vitrocéramique à base de silicate de lithium est utilisée, qui comporte les constituants suivants :
| Constituant | % en poids | |
|---|---|---|
| SiO₂ | 64,0 - 75,0, | de préférence 64,0 - 73,0 |
| Li₂O | 13,0 - 17,0 | |
| K₂O | 2,0 - 5,0 | |
| Al₂O₃ | 0,5 - 5,0 | |
| Agent de nucléation | 2,0 - 5,0 | |
| Me(II)O | 0 - 3,0 | |
et qui comporte moins de 0,1 % en poids de ZnO,
le Me(II)O étant sélectionné au moins parmi CaO, BaO, MgO et SrO,
et qui comporte du métasilicate de lithium en tant que phase cristalline principale.

2. Procédé selon la revendication 1, selon lequel la vitrocéramique est sensiblement exempte de ZnO.

3. Procédé selon la revendication 1 ou 2, dans lequel la vitrocéramique comporte 0 à 2,0 et de préférence 0 à 1,5 % en poids de Me(II)O).

4. Procédé selon l'une quelconque des revendications 1 à 3, dans lequel Me(II)O est sélectionné au moins parmi CaO et MgO.

5. Procédé selon l'une quelconque des revendications 1 à 4, dans lequel la vitrocéramique comporte 0,1 à 1,0 % en poids de MgO.

6. Procédé selon l'une quelconque des revendications 1 à 5, dans lequel le rapport molaire de SiO₂ : Li₂O est au moins de 2,2 : 1, de préférence au moins de 2,3 : 1, et est de manière particulièrement avantageuse compris dans la plage allant de 2,3 : 1 à 2,5 : 1.

7. Procédé selon l'une quelconque des revendications 1 à 6, dans lequel le rapport molaire de Al₂O₃ : K₂O est compris dans la plage allant de 1 : 0,5 à 1 : 2,0 et de préférence de 1 : 1 à 1 : 2,0.

8. Procédé selon l'une quelconque des revendications 1 à 7, dans lequel la vitrocéramique comporte 2,5 à 5,0 % en poids de Al₂O₃.

9. Procédé selon l'une quelconque des revendications 1 à 8, dans lequel la vitrocéramique comporte 70,0 à 73,0 % en poids de SiO₂.

10. Procédé selon l'une quelconque des revendications 1 à 9, dans lequel la vitrocéramique comporte 0 à 4,0, de préférence 0,1 à 4,0, notamment 1,0 à 4,0 et de manière particulièrement avantageuse 1,5 : 3 % en poids de ZrO₂.

11. Procédé selon l'une quelconque des revendications 1 à 10, dans lequel la vitrocéramique comporte au moins un des constituants suivants, avec les quantités suivantes :
| Constituant | % en poids | |
|---|---|---|
| Li₂O | 14,0 - 16,0 | |
| K₂O | 3,0 - 4,5 | |
| oxydes métalliques colorants et fluorescents | 0,5 - 7,5, | de préférence 0,5 à 3,5. |

12. Procédé selon l'une quelconque des revendications 1 à 11, dans lequel la vitrocéramique comporte en outre au moins un des constituants supplémentaires suivants :
| Constituant | % en poids |
|---|---|
| Na₂O | 0 - 2,0 |
| B₂O₃ | 0 - 5,0 |
| F | 0 - 5,0 |

13. Procédé selon l'une quelconque des revendications 1 à 12, dans lequel l'agent de nucléation est au moins un des suivants : P₂O₅ et des composés des éléments Pt, Ag, Cu et W.

14. Procédé selon l'une quelconque des revendications 1 à 13, dans lequel le métasilicate de lithium forme plus de 50 et jusqu'à 80 % en volume de la vitrocéramique à base de silicate de lithium.

15. Procédé selon l'une quelconque des revendications 1 à 14, dans lequel la vitrocéramique se présente sous la forme d'une ébauche.

16. Procédé selon l'une quelconque des revendications 1 à 15, dans lequel la restauration dentaire est un inlay, un onlay, un bridge, un pilier, une facette, une facette pelliculaire (veneer), une facette d'abrasion, une couronne, une couronne partielle, une infrastructure ou une coiffe.

17. Procédé selon l'une quelconque des revendications 1 à 16, dans lequel la restauration dentaire comprend du disilicate de lithium en tant que phase cristalline principale.

18. Procédé selon l'une quelconque des revendications 1 à 17, dans lequel la vitrocéramique à base de métasilicate de lithium est préparée par
(a) production d'un verre de départ contenant les constituants de la vitrocéramique;
(b) application d'un premier traitement thermique au verre de départ , à une première température, pour obtenir un produit de verre qui contient des germes aptes à la formation de cristaux de métasilicate de lithium;
(c) application d'un deuxième traitement thermique au produit de verre, à une deuxième température qui est plus élevée que la première température, pour obtenir la vitrocéramique à base de silicate de lithium, avec le métasilicate de lithium en tant que phase cristalline principale.

19. Procédé selon la revendication 18, dans lequel le premier traitement thermique de l'étape (b) comprend le chauffage du verre de départ jusqu'à une température de 500 à 600 °C, pour une durée allant d'environ 10 minutes à 3 heures.

20. Procédé selon la revendication 18 ou 19, dans lequel le deuxième traitement thermique de l'étape (c) comprend le chauffage du produit de verre jusqu'à une deuxième température de 680 à 720 °C, de préférence de 690 à 710 °C, et de manière particulièrement avantageuse d'environ 700 °C.

21. Procédé selon l'une quelconque des revendications 18 à 20, dans lequel le verre de départ de l'étape (a), le produit de verre de l'étape (b) ou la vitrocéramique à base de métasilicate de lithium de l'étape (c) est formé jusqu'à obtention d'une géométrie souhaitée, par usinage ou par compression à chaud.

22. Procédé selon la revendication 21, dans lequel l'usinage est réalisé par meulage, rognage ou fraisage.

23. Procédé selon l'une quelconque des revendications 18 à 22, qui comprend en outre l'application à la vitrocéramique à base de silicate de lithium de l'étape (c), d'un troisième traitement thermique, à une troisième température de 830 à 880 °C, pour une durée de 10 à 60 minutes.

24. Utilisation de la vitrocéramique définie dans l'une quelconque des revendications 1 à 15 pour la préparation d'une restauration dentaire.

25. Utilisation d'une vitrocéramique à base de silicate de lithium pour la préparation d'une restauration dentaire, où la vitrocéramique à base de silicate de lithium comprend les constituants suivants :
| Constituant | % en poids |
|---|---|
| SiO₂ | 64,0 - 73,0, |
| Li₂O | 13,0 - 17,0 |
| K₂O | 2,0 - 5,0 |
| Al₂O₃ | 0,5 - 5,0 |
| Agent de nucléation | 2,0 - 5,0 |
| Me(II)O | 0 - 1,5 |
où le rapport molaire de Al₂O₃ : K₂O est compris dans la plage allant de 1 : 0,5 à 1 : 2,0,
le Me(II)O étant sélectionné au moins parmi CaO, BaO, MgO et SrO,
et comporte moins de 0,1 % en poids de ZnO.

26. Utilisation d'un verre à base de silicate de lithium pour la préparation d'une restauration dentaire, où le verre à base de silicate de lithium comprend les constituants suivants :
| Constituant | % en poids |
|---|---|
| SiO₂ | 64,0 - 73,0, |
| Li₂O | 13,0 - 17,0 |
| K₂O | 2,0 - 5,0 |
| Al₂O₃ | 0,5 - 5,0 |
| Agent de nucléation | 2,0 - 5,0 |
| Me(II)O | 0 - 1,5 |
où le rapport molaire de Al₂O₃ : K₂O est compris dans la plage allant de 1 : 0,5 à 1 : 2,0,
le Me(II)O étant sélectionné au moins parmi CaO, BaO, MgO et SrO,
et comporte moins de 0,1 % en poids de ZnO, et comporte des germes aptes à la formation de métasilicate de lithium.

27. Utilisation selon la revendication 26, où le rapport molaire de SiO₂ : Li₂O est au moins de 2,2 : 1, de préférence au moins de 2,3 : 1, et est de manière particulièrement avantageuse compris dans la plage allant de 2,3 : 1 à 2,5 : 1.

28. Utilisation selon la revendication 26 ou 27, où le rapport molaire de Al₂O₃ : K₂O est compris dans la plage allant de 1 : 1 à 1 : 2,0.
